# EUROPEAN PATENT APPLICATION

(11) **EP 4 538 974 A1**
(43) Date of publication of application: **16.04.2025**
(21) Application number: 23315382.4
(22) Date of filing: 10.10.2023
(51) Int. Cl.: G06T 7/33, G06T 7/00, G06T 7/246, A61B 5/00

(54) **A COMPUTER IMPLEMENTED METHOD, A COMPUTER PROGRAM PRODUCT, A COMPUTER-READABLE STORAGE MEDIUM, A USE OF THE COMPUTER IMPLE-MENTED METHOD, AND A SYSTEM TO EXECUTE THE STEPS OF THE METHOD**

(71) Applicant: Caranx Medical, 75013 Paris (FR)
(72) Inventor: MIRA, Anna, 06000 Nice (FR); WEI, Wen, 06410 Biot (FR); BERTHET-RAYNE, Pierre, 06800 Cagnes-sur-Mer (FR); SMITS, Jonas Victor Harmen, 3360 Bierbeek (BE)
(74) Representative: Hepp Wenger Ryffel AG

(57) **Abstract**

The invention relates to a computer implemented method for de- . termining a position and/or an orientation of a valve component or a valve annulus component, preferably the annular ring (2) of a heart valve, in particular during an implantation of a heart valve prosthesis (3), comprising the steps of (i) receiving at least one first image (4) and at least one second image (5) of a moving heart via an input interface, in particular in real-time, (ii) identifying at least one first feature (7) indicative of a position and/or an orientation of the valve component or the valve annulus component and at least one second feature (8) in the at least one first image (4) using a data processing unit (6), (iii) determining at least one static or dynamic geometric relationship between the at least one first feature (7) and the at least one second feature (8) based on a localization of the first and second features (7, 8) in the at least one first image (4), using the data processing unit (6), identifying the at least one second feature in the at least one second image (5) using the data processing unit (6), (iv) determining a modified position and/or orientation (20) of the valve component or the valve annulus component in the at least one second image (5) based on the at least one second feature (8), the at least one static or dynamic geometric relationship, and a static or dynamic geometric relationship model, and (v) transmitting the modified position and/or orientation (20)-of the valve component or the valve annulus component to an output interface.

## Description

The present invention relates to a method for determining a position and/or an orientation of a valve component of a valve annulus component, preferably the annular ring of a heart valve, a computer program product, a computer-readable storage medium, a use of the of the computer implemented method, and a system to execute the steps of the method.

Cardiac surgery and in particular transcatheter aortic valve implantation or replacement (TAVI/TAVR) requires accurate localization of the native annular ring of a patient which serves at the anchoring point, e.g. for an aortic heart valve prosthesis.

The implantation procedure of an aortic heart valve prosthesis poses severe risks of medical complications which may lead to strokes or even death due to dislodgement of calcified material or clots, acute kidney injury/failure due to contrast agent administration, vascular dissection, and/or vascular hematoma or bleeding.

Misplacement of the aortic prosthetic valve may further lead to a plurality of adverse surgical outcomes resulting in valve migration, a need for a follow-up surgery or reposition of the aortic heart valve, an arrhythmia, an aortic regurgitation, e.g. paravalvular leaks due to incomplete sealing with the native tissue, a cardiac injury and/or even a coronary obstruction.

Different medical imaging methods such as Magnetic Resonance Imaging (MRI), Computer Tomography (CT), and fluoroscopy enable clinicians to determine regions of interest, e.g. the annular ring, for performing cardiac surgery, in particular TAVI/TAVR. However, accurately identifying and tracking anatomical structures such as the annular ring or calcifications, in particular together with medical instrument such as a delivery device and/or a prosthetic aortic heart valve, is influenced by the cardiac and respiratory cycle, blood flow and systolic/diastolic pressure. Furthermore, tracking of radiolucent anatomical structures is challenging since the imaging techniques such as fluoroscopy necessitate the administration of contrast agent for precise identification and tracking of these features.

EP 2 757 528 B1 relates to a method and device for tracking objects in a target area of a moving organ such as the heart which is subject of constant motion based on determining a dynamic geometric relationship between two features.

However, the prior art lacks a reliable computer implemented method for determining an orientation and/or position of a valve component or a valve annulus component, in particular the annular ring, a computer program product, a computer-readable storage medium, a system to execute the method. In particular, the prior art fails to precisely track the position/orientation of the annular ring and/or necessitates repeated administration of contrast agent.

In particular, the prior art fails to take-into- consideration the position and/or orientation of the valve component or valve annulus component during transient Rapid Ventricular Pacing (RVP). RVP is used for delivering rapid electrical impulses to the heart increasing the heartbeat rate, e.g. via an external pacemaker, creating a brief period of "cardiac standstill", which allows for positioning and deployment of an aortic heart valve prosthesis.

Moreover, there is a need in the prior art for simplifying crossing the annular ring with a prosthetic implant and positioning by providing a position and/or orientation of the annular ring, especially if rapid pacing is carried out during valve delivery.

The present invention adeptly addresses and provides solutions to the above-mentioned technical problems, and in particular provides a reliable computer implemented method, a computer program product, a computer-readable storage medium, use of the method, and system to execute the steps of the method for determining and transmitting a modified position/orientation of a valve component or a valve annulus component, preferably the annular ring. The valve component or valve annulus component may comprise or consist of the right coronary cusp, the left coronary cusp, the non-coronary cusp, the aortic anulus, the right aortic sinus, the left aortic sinus, the posterior aortic sinus, and/or the nodules of Arantius.

In a preferred embodiment, the position/orientation of the aortic annular ring is determined and transmitted. However, alternatively e.g. the orientation/position of the mitral, tricuspid, or pulmonary valve annulus or components thereof, in particular the leaflets/cusps, sinuses, or tendinous cords, may be determined and transmitted.

The invention shall further provide improved placement/orientation accuracy of a heart valve prosthesis with respect to the valve component or valve annulus component which is not dependent on education and training of clinicians, and reduce intra and/or postoperative clinical complications, e.g. valve migration, paravalvular leakage, prosthesis embolization, atrioventricular block, coronary (ostium) obstruction, hemolysis, valve malfunction, turbulent blood flow, hemodynamic instability, or dislodgement of vascular plaque. Furthermore, the invention enables a reduction of the procedural time and/or administration of contrast agent shall while allowing for a standardized procedure which minimizes human error and ensures consistent and repeatable patient outcomes. The invention shall further shorten the procedural time and decrease radiation exposure by fluoroscopic guidance of both the patient and the clinicians.

According to a first aspect of the invention, a computer implemented method for determining a position and/or an orientation of a valve component or a valve annulus component, preferably an annular ring, in particular during an implantation of a heart valve prosthesis is provided. The method comprises receiving at least one first image and at least one second image of a moving heart via an input interface, in particular in real time. The method comprises identifying at least one first feature indicative of a position and/or an orientation of the valve component or valve annulus component and at least one second feature in the at least one first image using a data processing unit. The method further comprises determining at least one static or dynamic geometric relationship between the at least one first features and the at least one second feature based on a localization of the first and second features in the at least one first image, using the data processing unit The method includes identifying the at least one second feature in the at least one second image using the data processing unit. The method further comprises determining a modified position and/or orientation of the valve component or valve annulus component in the at least one second image based on the at least one second feature, the at least one modified static or dynamic geometric relationship, and a static or dynamic geometric relationship model to account for the position and/or orientation of the valve component or valve annulus component, in particular during rapid pacing of the heart. The method includes transmitting the modified position and/or orientation of the valve component or valve annulus component to an output interface.

This enables to computationally integrate the determination of the valve component or valve annulus component position and/or orientation, e.g. for crossing the annular ring with a heart valve prosthesis, in particular during rapid pacing, and subsequent precise positioning/orienting of the heart valve prosthesis during deployment. Determining the modified orientation of the valve component or valve annulus component in addition to the modified position allows for a more accurate localization of the valve component or the valve annulus component, averting potential risks during deployment of a heart valve prosthesis in the annular ring.

The induced "quiescent" state of the heart by rapid pacing leads to the valve component or the valve annulus component position/orientation being affected differently as compared to a normal cardiac cycle. During rapid pacing the heart is beating repeatedly and quickly, e.g. between 180 and 220 induced beats per minute, transitioning through the systolic phase. This influences the position/orientation of the valve component or the valve annulus component, in particular the annular ring, significantly since the blood flow has a significantly decreased stroke volume and a transient drop in blood pressure, most noticeably systolic blood pressure.

The valve component or the valve annulus component position/orientation is subject to constant movement based on the respiratory and cardiac cycle. During rapid pacing for example, the aortic valve is predominantly open, and the position of the annular ring corresponds the systolic phase of a normal cardiac cycle while the respiratory cycle perpetually affects the position and/or orientation of the annular ring.

The heart valve prosthesis may be an aortic heart valve prosthesis which is typically used in TAVI/TAVR procedures.

The at least one first image may be a pre-operative CT image or a pre-operative MRI image. This enables the method to overlay the information such as first and second features of the image in three dimensions with the second image. The second image may be a fluoroscopy image which may be acquired intraoperatively, in particular in real-time. Fluoroscopy images during TAVI/TAVR are typically acquired at different orientations, e.g. a C-arm may have a cranial (CRA), caudal (Cau), left anterior oblique (LAO), or right anterior oblique (RAO) orientation. A three-dimensional CT image allows for tracking anatomical or procedural second and/or first features in the fluoroscopy images at different orientations to precisely locate the features.

Alternatively, the first image may be an intraoperative fluoroscopy image as well.

The first-feature indicative of the position/orientation of the valve component or valve annulus component may be formed by a calcification and/or vascular plaque, a procedural feature, e.g. a pigtail guidewire/catheter, or the valve component or valve annulus component itself which may be visible when administering contrast agent. In a preferred embodiment, the calcifications and/or vascular plaque on the native valve may form the first feature.

The at least one second feature may be a hypodense or hyperdense landmark. Hypodense landmarks may be fat deposits, cysts or fluid collections, or air or gas pockets. Hyperdense landmarks may be a calcification and/or vascular plaque, an implant or medical instrument, a bone structure, and/or a contrast agent.

The at least one first image may be a normal-flow image being acquired in the absence of rapid pacing and the second image may be a reduced-flow image acquired during rapid pacing.

The second image which is preferably acquired during rapid pacing provides a more accurate position of the second feature during actual rapid pacing which in turn allows to determine a more precise modified position and/or orientation of the valve component or the valve annulus component, in particular the annular ring. The heart valve prosthesis is typically positioned and deployed during rapid pacing such that a more precisely determined modified position/orientation may prevent adverse effects due to incorrect positioning/orienting of the heart valve prosthesis.

The method may comprise identifying the presence or the absence of at least one first feature indicative of a position and/or orientation of the valve component or the valve annulus component in the at least one second image. If the presence of the first feature was identified the modified position and/or orientation of the valve component or the valve annulus component in the at least one second image is determined based on the first feature. If the absence of the first features was identified, the modified position and/or orientation of the valve component or the valve annulus component in the at least one second image is determined based only on the at least one second feature and the at least one static or dynamic geometric relationship and the static or dynamic geometric relationship model.

This enables the method to selectively optimize the determined modified position and/or orientation of the valve component or the valve annulus component based on whether or not the first feature is visible in the at least one second image. Hence, if the presence of the first feature can be determined, e.g. based on fluoroscopy imaging in combinations with an administration of a contrast agent, the first feature indicative of the position and/or orientation of the valve component or the valve annulus component, e.g. during pacing, can be identified with greater precision.

Alternatively, the first feature may be formed by an anatomical landmark, e.g. calcifications which is at least partially visible without the use of a contrast agent. The first feature may be formed by a pre-implanted existing heart valve prosthesis which is visible without contrast agent under fluoroscopic imaging.

Instead, if the absence of the first feature is identified, the method still provides a reliable modified position and/or orientation of the valve component or the valve annulus component. This combination ensures that the modified position/orientation can be determined consistently and accurately irrespective of the presence/absence of the first feature.

The method may comprise determining a position and/or an orientation of a heart valve prosthesis and/or of a delivery device for delivering the heart valve prosthesis in at least one of the first and/or second images in real-time, in particular with respect to the modified position and/or orientation of the valve component or the valve annulus component.

By accurate determination of the position/orientation of the prosthesis or delivery device, a positional deviation with respect to the modified position/orientation of the valve component or the valve annulus component can be determined. This provides valuable data for crossing and deploying the heart valve prosthesis either manually by a clinician or by a partially or fully automated robotic system.

The first feature in the at least one first image may be indicative of a maximum radial dimension of the annular ring. The method may comprise the step of determining a corrected radial dimension of the annular ring in the at least one second image based on the maximum radial dimension. In addition to the maximum radial dimension, the target radial dimension may be determined based on a correction to account for a radial dimension of the annular ring, in particular during rapid pacing.

The maximum radial dimension may be determined based on a plurality of first images showing different radial dimensions of the annular ring during normal cardiac rhythm.

Since the annular ring is not a rigid structure its radial dimension changes during the cardiac cycle, i.e. during systole and diastole. Optimized placement of the heart valve prosthesis can be achieved via expanding the heart valve prosthesis corresponding to the maximum radial dimension.

Reliable determination of the target radial dimension in combination with the modified position and/or orientation of the annular ring is beneficial for selection and correct deployment of a heart valve prosthesis. Incorrect deployment of the heart valve prosthesis, i.e. incorrect expansion and/or alignment during deployment for example by a balloon catheter, may result in valve embolization, e.g. based on valve migration, conduction disturbances, coronary obstructions, paravalvular leakage/valve malposition, stroke, or tearing/rupture of the aortic annulus.

The at least one determined static or dynamic geometric relationship may be indicative of the cardiac cycle and/or the respiratory cycle of the patient. A first static or dynamic geometric relationship may be based on the cardiac cycle and a second static or dynamic geometric relationship may be based on the respiratory cycle.

A plurality of first images may be acquired, preferably in a continuous manner, to determine the dynamic geometric relationship of the cardiac and/or the respiratory cycle and in particular to discriminate between the cardiac and respiratory cycle. The cardiac and respiratory cycle typically both influence the movement of the valve component or the valve annulus component in an essentially periodic manner on different time scales, such that the method can be adapted to determine the influence of either based on the plurality of first images independently from each other.

The respiratory cycle may for example be determined based on a periodic movement of an anatomic or procedural feature, e.g. the respiratory movement of the diaphragm of a patient which displaces the heart. The cardiac cycle may be determined based on an electrocardiogram (ECG) and/or by the cardiac movement of an anatomic or procedural feature, e.g. a pigtail guidewire/ catheter.

This enables to determine the modified position/orientation of the valve component or the valve annulus component based on the dynamic geometric relationship(s) in a more reliable manner since the cardiac cycle changes, in particular during rapid pacing, while the respiratory cycle is continuously maintained.

The at least one second feature may be formed by the anatomical feature of the patient or the procedural feature, in particular a medical instrument or a pose of the medical instrument. One second feature may be an anatomical feature and another second feature may be a procedural feature.

Using a plurality of second features, in particular a plurality of anatomical and/or a plurality of procedural features, allows to enhance the precision and reliability and is less error-prone, e.g. to errors in identification of at least one of the second features. This may be advantageous, if certain second features are less visible/more visible during administration of contrast agent. For example, medical instruments are often configured to be radiopaque to be visible under fluoroscopy and thus less visible during administering of radiopaque contrast agent. Vice versa, many anatomic features are more visible under fluoroscopy during the administration of contrast agent.

The pose of a medical instrument may in particular relate to a position/orientation and/or a shape of a bent/curved guidewire/catheter, in particular a pigtail catheter/guidewire, known to the skilled person which was introduced into the ventricle or non-coronary cusp of a patient.

The pose of the bent/curved guidewire/catheter may assume a spiral shape. This pose may be continuously deformed during the cardiac cycle and/or the respiratory cycle and can be directly associated with characteristics of an ECG of the heart. Thus, the pose is particularly suited for determining a modified position/orientation of the valve component or the valve annulus component. The medical instrument or the deformation of the pose during pacing and during normal blood flow may be associated with different ECG characteristics.

In alternative or addition to a common pigtail catheter, a guidewire/catheter with a coiled/curved distal tip may be used which is particularly suited for TAVI/TAVR. The coiled/curved guidewire/catheter within this application may be formed by a commercially available Safari guidewire.

In a preferred embodiment, a first medical instrument comprising a pigtail catheter/guidewire is placed in the non-coronary cusp and/or a second medical instrument comprising a bent/coiled guidewire/catheter is placed in the left ventricle. Placing the pigtail catheter/guidewire in the non-coronary cusp of the aortic valve allows for optimized contrast injection, hemodynamic monitoring, deflecting medical instruments away from delicate structures, and may further assist with positioning of the aortic heart valve prosthesis. Placing the bent/curved guidewire/catheter in the left ventricle provides support for the system for positioning of the heart valve prosthesis and may serve as navigation aid to navigate the anatomy of the heart while also serving as an anatomic reference point.

The method may further be adapted to determine the modified position and/or orientation of the valve component or the valve annulus component without the administration of the contrast agent in any of the method steps. A contrast agent free method may curtail associated costs of the surgical procedure and optimize the operational workflow, rendering the solution economically and operationally more time efficient.

The method may comprise processing, preferably in real-time, the at least one first and/or second images and including at least one of (i) the modified position and/or orientation of the valve component or the valve annulus component and (ii) the position and/or orientation of the heart valve prosthesis in (I) a pre-operative visualization of the heart or (II) a computational model of the valve component or the valve annulus component, in particular a finite element model.

This allows to provide enhanced patient-specific visualization and more spatial information, in particular three-dimensional information, of the valve component or the valve annulus component and heart valve prosthesis for a clinician or a robotic control which facilitate performing a surgical procedure, in particular a TAVI/TAVR procedure. Moreover, this enables operative planning by providing information of the spatial relationship between the valve component or the valve annulus component and the heart valve prosthesis which is paramount for a complex surgery such as TAVI/TAVR and minimizes the potential for human error, ensuring consistent performance regardless of the clinician's expertise level.

The computational model may include at least one, in particular a plurality of static or dynamic geometric relationships. The at least one static or dynamic geometric relationship may be determined based on a determined respiratory or cardiac cycle, in particular a first static or dynamic geometric relationship based on the respiratory cycle and another static or dynamic geometric relationship based on -the cardiac cycle. These static or dynamic geometric relationships may be used as boundary conditions for the computational model.

The method may comprise displaying the modified position and/or orientation of the valve component or the valve annulus component and/or the heart valve prosthesis in (i) the at least one first image or the second image, (ii) the pre-operative visualization, and/or (iii) integrating it in the computational model.

This enables enhanced visualization by providing the dynamics of the valve component or the valve annulus component and/or the heart valve prosthesis which may assist clinicians carrying out a surgical procedure in a precise and accurate manner leading to safer surgical procedures, improved patient outcomes, and reduced procedural times.

The method may comprise receiving a user generated input, e.g. via a user interface, to adjust the modified position and/or orientation of the valve component or the valve annulus component, in particular the annular ring, manually and displaying the adjusted modified position and/or orientation instead of the prior modified position and/or orientation, preferably in real-time.

This allows a clinician to adjust the determined position/orientations which safeguards against potential computational or procedural errors by enabling clinicians to adjust the results, e.g. by interactively selecting a location or region in the image. Clinicians typically have tacit knowledge of anatomy, physiology and experience which may allow to catch outliers/errors which are not readily apparent from the computational data.

The method may comprise classifying the at least one first and/or second images to determine if the respective image was acquired under the influence of a contrast agent. The method may further comprise determining one of the at least one first feature and the second feature which is not visible without contrast agent based on the other one of the at least one first feature and the second feature which is visible without contrast agent if the image was not acquired under the influence of the contrast agent.

This allows to reliably determine the features which are not or not easily visible under the influence of contrast agent, e.g. radiopaque medical instrument or calcifications, or are not or not easily visible without the influence of contrast agent, e.g. many native anatomic structures such as the aortic arch or the annular ring.

The-step of determining the modified position and/or orientation of the valve component or the valve annulus component may be based on an ECG of the heart using the data processing unit.

Using an ECG in addition to the static or dynamic geometric relationship to determine the modified position and/or orientation of the valve component or the valve annulus component allows for bias corrections and, thus, determining the modified position/orientation in a more accurate manner.

Another aspect of the invention relates to a computer program product comprising instructions which, when the program is executed by a computer, cause the computer to carry out the steps of the previously described method.

A further aspect of the invention relates to a computer-readable storage medium comprising instructions which, when executed by a computer, cause the computer to carry out the steps of the previously described method.

Another aspect of the invention relates to a use of the previously described computer implemented method for at least one of (i) planning a transcatheter aortic valve implantation or replacement, (ii) translationally moving, rotationally moving, and/or tilting about a longitudinal axis at least one medical instrument, in particular a delivery system of a heart valve prosthesis for navigating and/or deploying the heart valve prosthesis, and (iii) optimizing an amount of contrast agent and/or a time interval of introducing the contrast agent into a body duct.

Translationally and rotationally moving and tilting the medical instrument about its longitudinal axis enables an optimized multidimensional adjustability of the medical instrument with respect to the modified position/orientation of the valve component or the valve annulus component. Hence, the medical instrument may be adjusted by a clinician to entirely different modified positions/orientations of the valve component or the valve annulus component during pacing to account for the patient-specific anatomy. This may provide additional utility for asymmetric heart valve prostheses and alignment with a typically asymmetric valve component or the valve annulus component which may have a spheric or ovoid shape. In addition, this may allow for optimized navigation/deployment to prevent a potential obstruction of the left or right coronary artery by the prosthesis.

Optimizing the amount of contrast agent/time interval of introducing contrast agent reduces costs while increasing workflow efficacy and further prevents adverse effects of contrast agent administration such as contrast-induced nephropathy.

Another aspect of the invention relates to a system comprising means, and in particular a data processing unit, adapted to execute the steps of the previously described computer implemented method.

The data processing unit may be configured for determining a dynamic radial dimension of the heart valve prosthesis or of an expansion unit for expanding the heart valve prosthesis, in particular comprising a balloon, in real time. The data processing unit is configured to provide (i) an expansion control command for operating the expansion unit or (ii) expansion control instructions to a user to operate the expansion unit, for radially expanding the heart valve prosthesis in the annular ring, in particular until reaching at least the corrected radial dimension of the annular ring.

This expansion of the heart valve prosthesis can be monitored/operated by clinicians without a steep learning curve in an efficient and time saving manner. Alternatively, the expansion may be carried out in a partially or fully automated manner. By rendering the expansion of the prosthesis more controllable the previously mentioned adverse effects of incorrect deployment of the heart valve prosthesis can be averted.

The system, in particular the data processing unit of the system, may comprise a user interface which allows a user to enter a user input. The user input may comprise (i) a selected type of the heart valve prosthesis, (ii) a target location and/or orientation of the selected heart valve prosthesis with respect to the valve component or the valve annulus component, in particular the annular ring, and/or (iii) an adjusted modified position/orientation of the valve component or the valve annulus component, in particular the annular ring. The system, in particular the data processing unit, may be adapted to determine, and preferably provide data to display, the target location/orientation based on the user input and the modified position/orientation of the valve component or the valve annulus component.

The data processing unit may be configured for determining a deployment control command based on the modified position and/or the orientation of the valve component or the valve annulus component and the position and/or the orientation of the heart valve prosthesis for operating the delivery device, to deploy the heart valve prosthesis in the annular ring. The control command provides instructions on at least one of, in particular all of (i) adjusting a tilt of the heart valve prosthesis with respect to the annular ring, (ii) adjusting a circumferential orientation of the heart valve prosthesis with respect to the annular ring, and (iii) adjusting a longitudinal position of the heart valve prosthesis.

This provides guidance for accurate navigation and deployment of the heart valve prosthesis and minimizes the risk for human error.

The system may comprise a pacing unit which is configured for delivering pacing signals to a target region of a patient's heart in proximity of the valve component or the valve annulus component for reducing the flow across the valve. The pacing unit may be operable by the data processing unit.

Thereby an all-in-one system can be provided which integrates rapid pacing into one medical system which streamlines the procedural time, e.g. required for switching between different medical devices.

The pacing unit may be arranged on a catheter/a guidewire, in particular on a bent/curved catheter/guidewire such as the Safari guidewire/catheter. This allows the pacing unit to be at least partially inserted into the heart, in particular the left ventricle of the heart, for rapid pacing of the heart, e.g. at or above 180 - 220 beats per minute.

However, the pacing unit may also be arranged on a separate medical instrument which is adapted to be introduced into the right ventricle, e.g. via the femoral vein in the groin across the right atrium, for rapid pacing known to the person skilled in the art.

The pacing unit may be connected or connectable to an extracorporeal pacemaker. This allows the pacing unit to be readily disposable after performing the medical procedure.

The data processing unit may be configured for receiving at least one post-deployment image after deployment of the heart valve prosthesis and -identifying and validating the position and/or orientation of the heart valve prosthesis with respect to the first feature indicative of the position and/or orientation of the valve component or the valve annulus component in the at least one first image.

This enables for immediate corrective actions if the heart valve prosthesis is malpositioned to reduce the risk of complications.

By validating the position/orientation of the heart valve prosthesis with respect to the first feature, in particular during normal flow, i.e. without rapid pacing, which was acquired prior to implantation, differences indicating malpositioning which may lead to the previously mentioned adverse effects such as valve migration or paravalvular leakage can be identified.

The data processing unit may be configured for (i) controlling an imaging device positioning unit for positioning and/or orienting the imaging device or (ii) providing user instructions on how to operate the imaging device positioning unit. The imaging device positioning unit may comprise or consist of a C-arm.

The integrated coordination between the data processing unit and the imaging device positioning unit facilitates executing the method steps in a precise and efficient manner to optimize procedural outcomes and streamline the method.

The imaging device may an extracorporeal imaging device, in particular an extracorporeal X-ray imaging device, such as a C-arm. Alternatively, the imaging device may an intracorporeal imaging device, in particular an intracorporeal imaging device adapted for intrathoracic imaging, in particular attached to the delivery device of the heart valve prosthesis. The imaging device may alternatively be a transoesophageal echocardiography (TEE) imaging device.

Another aspect of the invention relates to a system, in particular a system as previously described, for guiding the placement of a heart valve prosthesis within an annular ring. The system comprises a data processing unit which is adapted for displaying on a display device in real-time a position and/or orientation of a heart valve prosthesis with respect to a modified orientation and/or position of the valve component or the valve annulus component in the reduced-flow image.

The modified position and/or orientation of the valve component or the valve annulus component may be based on an orientation of the annular ring that was corrected to account for a position and/or orientation of the annular ring during rapid pacing.

The system, in particular a data processing unit of the system may be adapted to determine the modified position and/or orientation in a previously described manner, i.e. based on the static or dynamic geometric relationship and the identified second feature in the second image.

The data processing unit may be adapted for generating visual markings, in particular color-coded markings, indicating the relative position and/or orientation of the heart valve prosthesis and/or instructions on reaching the desired orientation and/or position of the heart valve prosthesis in the annular ring in real-time. The visual markings or instructions may be based on at least one of, in particular all of, (i) a relative tilt of the orientation of the heart valve prosthesis with respect to the modified orientation of the annular ring, (ii) a relative circumferential orientation of the orientation of the heart valve prosthesis with respect to the modified orientation of the annular ring, and (iii)a relative--longitudinal position of the heart valve prosthesis with respect to the modified position of the annular ring.

Further embodiments of the invention and improvements of the described embodiments will become apparent with the following description of embodiments.

The invention is now described with reference to certain embodiments and figures which show:
Figures 1A and 1B: fluoroscopic images of a heart in the absence of rapid pacing during a TAVI/TAVR procedure during systole and diastole,
Figures 2A and 2B: fluoroscopic images of a heart during a TAVI/TAVR procedure during rapid pacing,
Figures 3A and 3B: a CT image and a fluoroscopic image of a heart during a TAVI/TAVR procedure,
Figures 4A and 4B: two augmented fluoroscopic images of a heart during a TAVI/TAVR procedure,
Figures 5A and 5B: a fluoroscopic image acquired of a heart acquired in an absence of rapid pacing during a TAVI/TAVR procedure and an enlarged section of the image including visual indicators,
Figures 6A and 6B: fluoroscopic images of a heart during a TAVI/TAVR procedure during rapid pacing in which the heart valve prosthesis has a collapsed state and the heart valve prosthesis assumes a partially expanded state, respectively,
Figure 7: a three-dimensional model of the of an aortic arch including the annular ring which was registered with a fluoroscopic image during TAVI/TAVR,
Figure 8A: a first CT image including a schematic representation of a pigtail catheter deployed in the non-coronary cusp of a patient,
Figure 8B: a second CT image including a schematic representation of a coiled/curved guidewire deployed in the left ventricle of the patient,
Figure 8C: a fluoroscopic image showing the deployed pigtail catheter and the deployed bent/curved guidewire according to Figs. 8A and 8B,
Figures 9A and 9B: a combined three-dimensional computational model based on a CT image and at least two fluoroscopic images acquired at different orientations,
Figure 9C: a fluoroscopic image including the information of the three-dimensional computational model of Figs. 9A and 9B,
Figure 9D: a schematic representation of a system including a data processing unit for carrying out the method according to the invention,
Figures 10A and 10B: a virtual schematic two-dimensional representation of the heart valve prosthesis and the aortic annulus having a visual marker which is indicative of a circumferential orientation of the heart valve prosthesis and a visual marker which is indicative of a circumferential orientation of the annular ring,
Figure 10C: a schematic three-dimensional representation of the heart valve prosthesis and the aortic root which indicates a tilt of the heart valve prosthesis with respect to the annular ring.

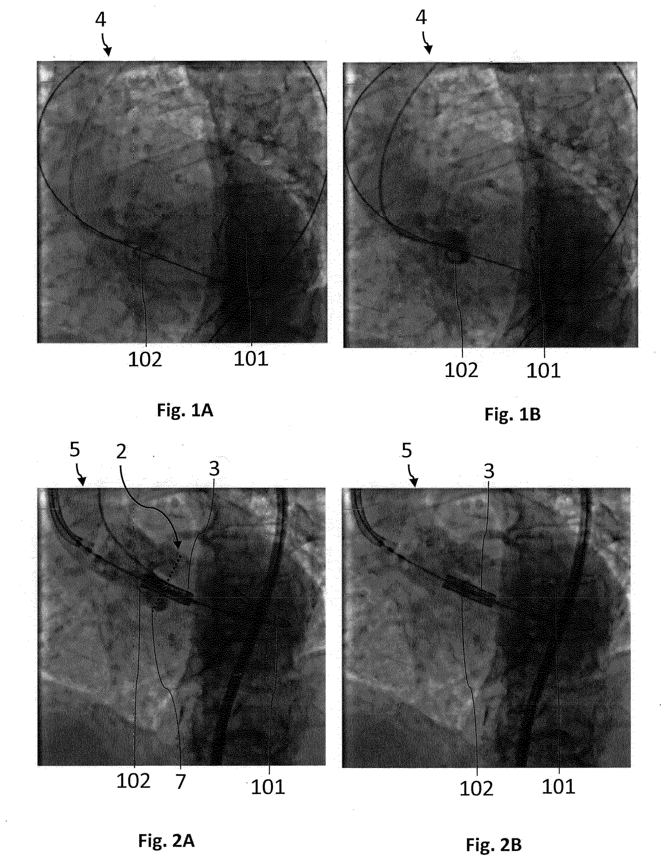

Figures 1A and 1B show a fluoroscopic first image 4 of a heart during a TAVI/TAVR procedure during the diastole and systole, respectively.

The average heart rate is around 60 - 80 beats per minute. During normal heart function, the sinoatrial (SA) node initiates a contraction of the atria and then ventricles via an electric signal for normal blood flow. Hence, an ECG typically shows a predictable pattern comprising of a P, QRS, and T wave. The P wave corresponds to atrial contraction, the QRS complex represents ventricular contraction, and the T wave shows ventricular recovery. This cardiac cycle can be monitored via an ECG of the system.

Figs. 1A and 1B show a medical instrument which comprises a Safari guidewire 101 and a pigtail catheter 102. The pigtail catheter 102 was advanced through the aorta and placed in the non-coronary cusp of the aortic arch of a patient. The Safari guidewire 101 was manipulated across the valve and into the left ventricle to provide support for the TAVI/TAVR procedure, i.e. to advance a heart valve prosthesis over the Safari guidewire 101.

Figs. 1A and 1B further show that the Safari guidewire 101 which contacts or is placed in proximity of the left ventricular apex of the heart is frequently deformed during normal cardiac function. Fig. 1A shows the heart during diastole in which the left ventricle relaxes and fills with blood. Fig. 1B shows the heart during systole in which the ventricular wall come together exerting force on the Safari guidewire.

This deformation of the shape of the medical instruments, in particular the Safari guidewire 101, and a first feature, e.g. the pigtail catheter 102, can be monitored during the normal cardiac cycle to determine a dynamic geometric relationship between the Safari guidewire and the first feature. The position and/or orientation the pigtail catheter 102, is directly indicative of the position/orientation of the annular ring adjacent the non-coronary cusp. Alternatively or additionally, a/the dynamic geometric relationship may be determined by monitoring the movement of the Safari guidewire 101,-and a different first feature than a pigtail catheter 102, such as e.g. a calcification in proximity of the annular ring. Furthermore, contrast agent may be administered such that a dynamic behaviour of the annular ring and the Safari guidewire or an anatomical feature may be monitored directly to determine the static or dynamic geometric relationship.

Based on a mathematical model, e.g. a pre-trained machine-learning model, a static or dynamic geometric relationship can be determined which accounts for the position and/or the orientation of the annular ring during rapid pacing of the heart. Hence, this model is designated as a static or dynamic geometric relationship model.

The machine-learning model, in particular a convoluted neural network, is preferably trained using fluoroscopic images of a patient population acquired during the normal cardiac cycle and fluoroscopic images acquired during rapid pacing. These training/validation images may be acquired during administration of contrast agent such that the position and/or orientation of the annular ring can be exactly determined in both images for the most reliable results. For ground truth labelling, the positions/ orientations of the annular ring and first and second features may be labelled, e.g. manually by a clinician or in an automated manner, such that these ground truths can be used during training and validation. The correct position/orientation of the annular ring in an absence of rapid pacing and during rapid pacing and position/orientation of the first and second features are used for training and validating the model in a manner known to a person skilled in the art.

In a preferred embodiment, the training images include anatomical and/or procedural features as first and second features. The model is preferably adapted to track the static or dynamic geometric relationship between these features such that the modified position/orientation of the annular ring can be determined based merely on these static or dynamic geometric relationship(s). This enables the model to be trained to associate the static/dynamic geometric relationship acquired during the normal cardiac cycle and determine the modified position/orientation the annular ring assumes/will assume during rapid pacing.

Based on a plurality of first fluoroscopic images acquired during the normal cardiac cycle the dynamic geometric relationship between the first feature/annular ring and a second feature is determined. This dynamic geometric relationship and the position/orientation of the annular ring are then input in the model to determine the modified position and/or orientation of the annular ring during rapid pacing, even when the first feature indicative of the annular ring position/orientation is non-visible.

Figures 2A and 2B show fluoroscopic images 5 of the heart during a TAVI/TAVR procedure during rapid pacing. Figures 2A and 2B further show a delivery device with a mounted heart valve prosthesis 3 which was advanced through the annular ring 2 during rapid pacing for deployment. A pigtail catheter 102 and a Safari guidewire 101 were placed analogously to Figs. 1A and 1B. Rapid pacing is typically carried out at a rate of 180 to 220 beats per minute such that the blood flow and pressure is reduced which allows for a more controlled environment for deploying an aortic heart valve prosthesis 3 which reduces the risk of valve migration, embolization, or malpositioning during deployment. During rapid pacing the diastole phase is significantly shortened such that the ventricles do not fill up completely in contrast to a normal heart rhythm/blood flow. A somewhat abbreviated systole phase is exemplary shown in Fig. 2A and a noticeably temporally shortened diastole phase is shown in Fig. 2B. A position of the annular ring 2 is visible in Fig. 2A based on the administration of a contrast agent. The administration of the contrast agent enables the position of the annular ring 2 to be tracked directly as the first feature 7 indicated by the dashed black line. As a second feature a pose of the Safari guidewire 101 which is continuously deformed in the absence and during rapid pacing is monitored.

The position of the annular ring 2 assumes a contracted state during rapid pacing more frequently and to a different degree and thus has a different position/orientation than during a normal cardiac cycle based on a reduced blood flow and cardiac pressure. A plurality of fluoroscopic images 5 similar to Figs. 2A and 2B may be used for training/validating the previously described machine learning model.

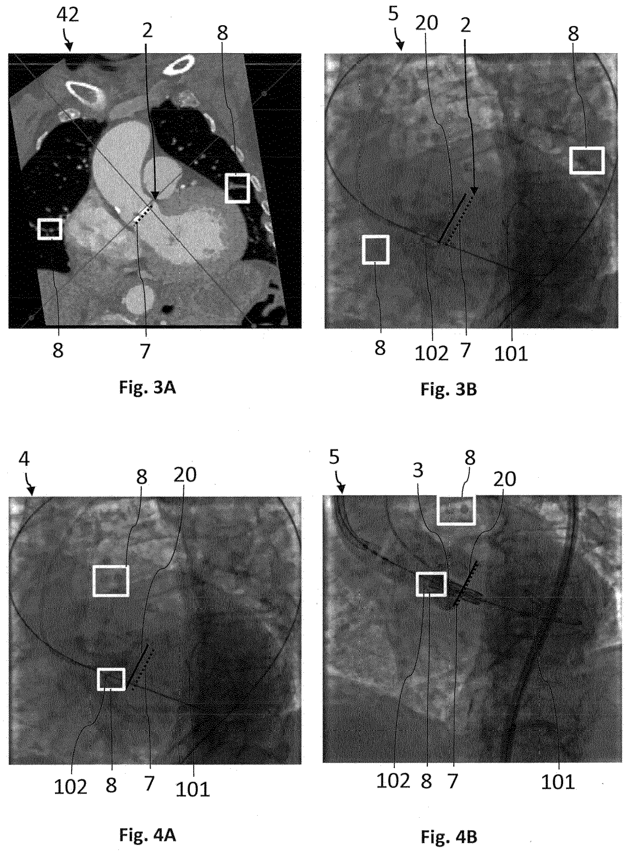

Figures 3A and 3B show a CT image 42 and a fluoroscopic image 5 of a heart during a TAVI/TAVR procedure with highlighted second features 8 and a highlighted first feature 7 indicative of a position and/or orientation of the annular ring 2.

Fig. 3A shows that the second features 8 and the first feature 7 are identified in the CT image 42 such that a static geometric relationship can be determined via the identified features 7, 8.

The second features 8 in Figs. 3A and 3B are indicated by the white boxes and the first feature 7 by the dashed black line.

The second features 8 are formed by hypodense landmarks in the CT image 4. The first feature 7 is formed by the annular ring 2 visible in the CT image 42. The CT image 42 in Fig. 3A was acquired under normal- blood flow. The CT image 42 allows to localize the features 7, 8 and, hence, a position/orientation of the annular ring 2 in three dimensions.

Fig. 3B shows that the same second features 8 are also determined in the second fluoroscopic image 5 based on feature matching the of second features 8 of the first CT image 42. The first feature 7 indicative of the position of the annular ring 2 is not visible in the second fluoroscopic image 5. The absence of the first feature 7 is detected and its position/orientation is determined and projected into the second image 5 based on the static geometric relationship between the identified features 7, 8. The first feature 7 is indicated by the dashed black line in Fig. 3B.

The second features 8 are used to change a position/an orientation of an imaging device, e.g. a C-arm, for deployment of a heart valve prosthesis.

However, a position/orientation of the annular ring 2 during rapid pacing which reduces the blood flow deviates from this determined position of the annular ring during normal blood flow.

A machine-learning model trained by a plurality of images acquired during normal blood flow and reduced blood flow during rapid pacing is used to counteract this bias. The model is configured to correct the determined static geometric relationship to account for the position/orientation of the annular ring during rapid pacing and determine a modified position and/or orientation 20 of the annular ring 2 based on this static geometric relationship shown in Fig. 3B.

Figs. 3A and 3B show that the modified position and/or orientation 20 is displayed within the CT or fluoroscopic image 4, 5 to provide guidance for a navigation and a deployment of a heart valve prosthesis via a pigtail catheter 102 and a Safari catheter 101.

Figures 4A and 4B show two fluoroscopic images 4, 5 of a heart during a TAVI/TAVR procedure with highlighted second features 8 and a first feature 7 indicative of a position/orientation of the annular ring 2 similarly to Figs. 3A and 3B. The first fluoroscopic image 4 was acquired in an absence of rapid pacing and the second fluoroscopic image 5 was acquired during rapid pacing. The figures 4A and 4B show that a modified position/orientation 20 of the annular ring 2 is determined based on correcting a static geometric relationship between the first feature 7 indicative of a position/orientation of the annular ring 2 and two second features 8 to account for rapid pacing. To illustrate that the modified position/orientation 20 of the annular ring differs in the absence of pacing and during pacing, the modified position/orientation 20 was included in both Figures 4A and 4B.

A second feature 8 in Figs. 4A and 4B indicated by the larger white rectangle is formed by a hypodense anatomical landmark. The first and/or second feature(s) 7, 8 are preferably selected in a partially or fully autonomous manner to alleviate a clinician's workload. Another second feature 8 indicated by the smaller white rectangle in Figs. 4A and 4B is a procedural feature formed by a pigtail catheter 102. A Safari guidewire 101 was further deployed in the left ventricle for supporting and guiding the crossing of the annulus via a heart valve prosthesis 3 and a deployment of the prosthesis 3.

In a preferred embodiment, a plurality of first fluoroscopic images 4 are used to determine a dynamic geometric relationship between the first feature 7 and the second features 8. The plurality of first images 4 is preferably used to discriminate between the influence of the respiratory cycle and the cardiac cycle on the movement of the features 7, 8. This improves the accuracy and reliability of the modified position/orientation 20 of the annular ring 2 during rapid pacing. The influence of the respiratory cycle remains essentially the same during rapid pacing or in absence of rapid pacing while the cardiac cycle changes considerably. The modified position/orientation 20 is preferably directly displayed within the intra-operative fluoroscopic image 5 in real-time.

Alternatively, a dynamic/static geometric relationship between two or more anatomic features similar to Figs. 3A and 3B or between two or more procedural feature is tracked to determine the modified position/orientation 20.

In a preferred embodiment, the dynamic geometric relationship between a Safari guidewire 101 introduced into the left ventricle is temporally tracked with respect to the position of the pigtail catheter 102 placed in the non-coronary cusp, in particular together with an ECG. These procedural features formed by medical instruments are particularly informative in predicting the movement of the annular ring 2, due to the close proximity to the annular ring 2 and their direct correlation with cardiac function.

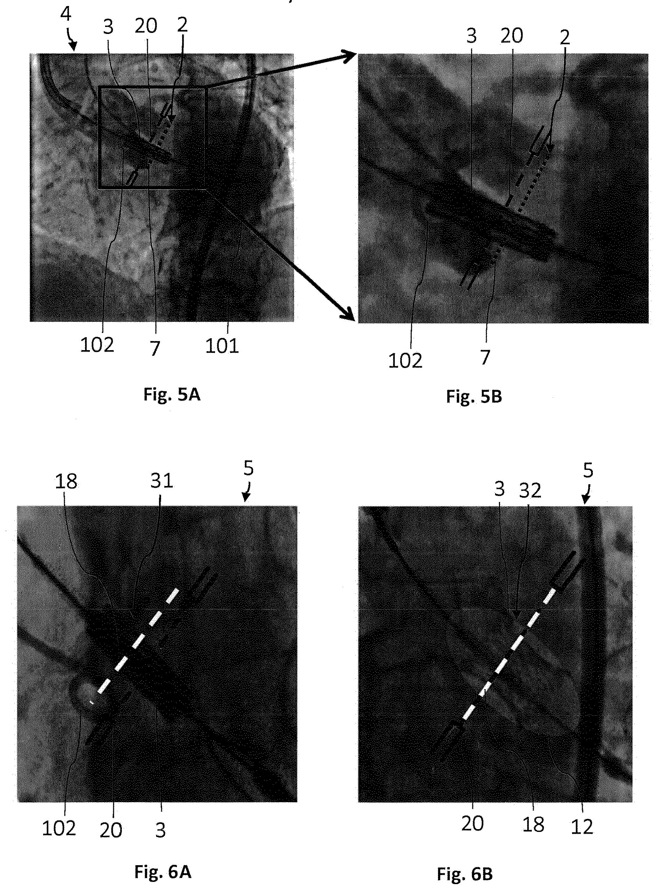

Figure 4B further shows that a delivery device for delivering a heart valve prosthesis 3 was introduced through the aortic arch. In a preferred embodiment, the position/an orientation of a heart valve prosthesis 3 (see Fig. 4A) in the images 4, 5 is tracked simultaneously with respect to the modified position/orientation 20 to support navigation/deployment of the heart valve prosthesis 3 and reduce the workload on clinicians (see Figs. 5A - 6B).

Figures 5A and 5B show a fluoroscopic image 4 in absence of rapid pacing during a TAVI/TAVR procedure and an enlarged section of the image including a modified position/orientation of the annular ring 20 and an annular ring 2 position/orientation.

Figures 5A and 5B show that a delivery device carrying an aortic heart valve prosthesis 3 was advanced through the aortic arch and a pigtail catheter 102 was placed in the non-coronary cusp and a Safari guidewire 101 was placed in the left ventricle.

The position of the first feature 7 indicative of a position/an orientation annular ring 2 in Figs. 5A and 5B is determined in a previously described manner, e.g. by tracking second features formed by anatomical and/or procedural features. Similarly, the machine-learning model is used for determining the modified position/orientation 20 of the annular ring 2.

Figures 5A and 5B show that the modified position/orientation 20 which was modified to account for rapid pacing is displaced in an antegrade direction with respect the position/orientation of the annular ring 2 during normal flow in the absence of rapid pacing. The fluoroscopic image 4 was acquired during an administration of a radiopaque contrast agent via the pigtail catheter 102 such that the annular ring 2 and the aortic arch are visible and can be tracked reliably. However, by providing the modified position/orientation 20 of the annular ring 2 during pacing, an additional administration of contrast agent during rapid pacing can be avoided.

In a preferred embodiment, the method includes determining, if the first feature 7 formed by the annular ring 2 is directly visible, e.g. due to the administration of the contrast agent, or if the first feature 7 is non-visible. If the presence of the first feature is identified, the method comprises determining the modified position/orientation 20 based on the identified annular ring 2. Otherwise, the method is determined via a static or geometric relationship between the first feature 7 and at least one second feature as previously described.

Figures 5A and 5B further show that in addition to the modified position/orientation 20, the method includes determining the maximum radial dimension of the annular ring 2 during the absence of rapid pacing. The maximum radial dimension in Figs. 5A and 5B is depicted as the length of the dashed line denoting the first feature 7 indicative of the annular ring 2.

The method is alternatively carried out merely based on tracking the pigtail catheter 102 and the Safari guidewire 101 as first and second features to determine the position/orientation of the annular ring 2 and the modified position/orientation 20 of the annular ring 2. Both the pigtail catheter 102 and the Safari guidewire 101 are visible without using contrast agent. This reduces the need for the administration of any contrast agent.

Figure 6A and 6B show fluoroscopic images 4 of a heart during a TAVI/TAVR procedure during rapid pacing in which a heart valve prosthesis 3 has a collapsed state 31 and the heart valve prosthesis assumes a partially expanded state 32, respectively.

A delivery device with the mounted heart valve prosthesis 3 was guided via fluoroscopy transfemorally through the aortic arch to the aortic annulus. A pigtail catheter 102 was placed in the non-coronary cusp and a Safari guidewire was placed in the left ventricle. A pacing wire is inserted through the right femoral vein and advanced into the right ventricle of the heart to deliver rapid pacing (not shown in Figs. 6A and 6B) for crossing the annular ring via the heart valve prosthesis 3.

Once the delivery device was introduced, a guidance software for carrying out the computer implemented method is activated. A distal tip of the pigtail catheter 102 is detected and tracked in a plurality of fluoroscopic images in absence of rapid pacing. The annular ring is detected and tracked as well in these images and a dynamic geometric relationship between the pigtail catheter 102 forming the second feature and the annular ring 2 forming the first feature is determined. The dynamic geometric relationship is modified to account for the displacement of the annular ring due to pressure changes and contractions during rapid pacing based on a dynamic geometric relationship model. This modification is carried out by a pre-trained machine-learning model which receives the position/orientation of the second feature and the dynamic geometric relationship as an input. A C-arm for acquiring the fluoroscopic images may be arranged in a single known orientation, e.g. in a standard 2-cusp view during the detection and tracking of these features.

The method includes displaying the fluoroscopic images 5 together with the modified position/orientation 20 of the annular ring during pacing which is not visible in the fluoroscopic images 5 of Figs. 6A and 6B.

The system for carrying out the method further provides a user interface in which the type of valve can be selected, and a target implantation depth 18 of the selected heart valve prosthesis 3 may be determined, e.g. based on a lookup table in a fully automated manner.

The user interface is adapted to receive a user input to correct the target valve implantation depth, e.g. by selecting a spatial position/orientation on a display device.

The figures 6A and 6B further show that the prosthetic heart valve prosthesis 3 is detected and tracked in the fluoroscopy images 5. The valve-specific target valve implantation depth 18 which is dependent on a valve prosthesis position/orientation is displayed in the fluoroscopic images 5 in real-time indicated by the white dashed line. This facilitates alignment of the valve implantation depth 18 and the modified position/orientation 20 of the annular ring by a clinician or in a fully automated manner. The system may optionally comprise instructions on how to best achieve the alignment.

In a preferred embodiment shown in Fig. 6B, the delivery device has an expansion unit which is formed by a balloon catheter 12 which is made of nylon. The balloon catheter 12 is radiolucent such that it is not visible under fluoroscopy. However, a partly deployed balloon catheter 12 was marked by a light grey elliptical region to represent a partially deployed balloon catheter 12.

The expansion state of the balloon, e.g. 75% in Fig. 6B, is detected and displayed on a display by the system such that the clinician or a robotic controller can precisely control the expansion state. The expansion state of the balloon may be detected by using radiopaque fluid for expanding the balloon or monitoring the expansion of the heart valve prosthesis 3 which is typically radiopaque. To further guide the expansion of the heart valve prosthesis 3, the dashed black line in Fig. 6B showing the modified position/orientation 20 of the annular ring also displays a target radial dimension of the annular ring indicated by its longitudinal length. The target radial dimension of the annular ring was determined based on the maximum radial dimension of the annular ring which was monitored in the fluoroscopic images during normal cardiac blood flow (see Figs. 5A and 5B) .

After successful deployment of the heart valve prosthesis 3 with the balloon catheter 12, the system validates a deployed position/orientation of the heart valve prosthesis 3. The valve position/orientation may be validated by detecting and tracking the heart valve prosthesis in the absence of rapid pacing in post-deployment fluoroscopic images and e.g. comparing the position/orientation with respect to the position/orientation of the annular ring. Subsequently, the delivery device can be removed.

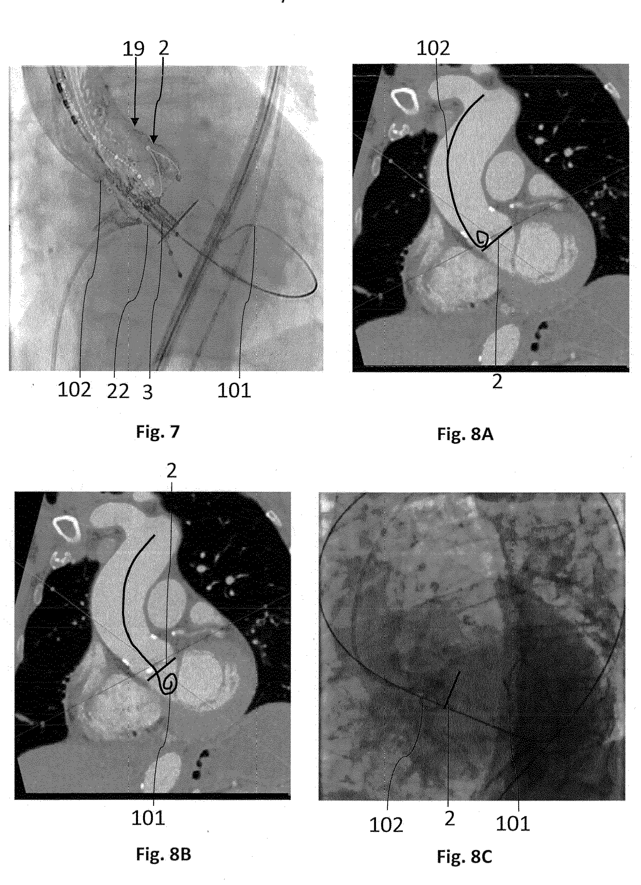

Figure 7 shows a three-dimensional model 19 of an aortic arch including the annular ring 2 which was registered with a two-dimensional fluoroscopic image during TAVI/TAVR. The fluoroscopic image includes a delivery device on which a heart valve prosthesis 3 is mounted and a deployed pigtail catheter 102 and a deployed Safari guidewire 101. A pacing wire 22 was introduced in the left ventricle to deliver rapid pacing during crossing of the annular ring 2. A contrast agent can be injected via the pigtail catheter 102 for detection and tracking of the annular ring during the procedure.

Subsequently, a software for guiding and tracking can be activated. The three-dimensional model 19 of the patient shown in Fig. 7 is generated and then registered with the two-dimensional fluoroscopic images which are intra-operatively acquired in real time during the administration of contrast agent. The three-dimensional model 19 includes the annular ring 2, hinge points and commissures and their corresponding three-dimensional positions/orientations. In a preferred embodiment, the three-dimensional model 19 includes the position of the right coronary ostium, the left coronary ostium, and/or the left non-coronary, the right and left, and non-coronary and right commissural regions between the cusps. A user is provided with instructions by the software on how to optimize a projection view of the fluoroscopic images and the user or a robotic controller manipulates the C-arm for acquiring the fluoroscopic images to achieve the optimal projection.

Figure 7 further shows that a position/orientation of the heart valve prosthesis 3 is detected and tracked in real time and included in the model 19. The user may adjust a target implantation depth (see Figs. 6A and 6B) of the heart valve prosthesis 3 via a user interface. The software determines and displays a modified position/orientation of the annular ring during pacing and a target implantation depth as previously described (see Figs. 6A and 6B) in the three-dimensional model 19 (not shown in Fig. 7). Alternatively, the three-dimensional model 19 is corrected to account for rapid pacing. In a further alternative, the three-dimensional model 19 is registered with fluoroscopic images acquired during rapid pacing of the heart. Based on the registration of the three-dimensional model 19 with the fluoroscopic images, a more exact position/orientation of the annular ring with respect to a target implantation depth of the heart valve prosthesis 3 is determined by the system. In particular a circumferential position of the heart valve prosthesis 3 with respect to the modified position/orientation of the annular ring during rapid pacing can be determined. A tilt of a longitudinal axis of the heart valve prosthesis 3 with respect to a radial orientation of the annular ring can also be included in the three-dimensional model 19 to allow a more precise deployment.

A user may further adjust the modified position/orientation of the annular ring 2 via a user input. This allows to overcome/resolve potential computation or procedural errors based on the professional expertise of clinicians. The target implantation depth is then adjusted accordingly by the system and displayed on a display. In a preferred embodiment, the system is further adapted to detect an expansion state of the heart valve prosthesis 3, provide guidance during a deployment of the heart valve prosthesis 3 via a balloon catheter and to carry out a validation of the deployment of the heart valve prosthesis 3 as described in Fig. 6B.

Figure 8A shows a first CT image including a schematic representation of a pigtail catheter 102 which was deployed in the non-coronary cusp of a patient. Figure 8B shows a second CT image including a schematic representation of a curved/coiled guidewire 101 deployed in the left ventricle of the patient. Figure 8C shows a fluoroscopic image showing a deployed pigtail catheter 102 and a deployed bent/curved guidewire 101 according to Figs. 8A and 8B. A position of the annular ring 2 was included in Figs. 8A - 8C for illustrative purposes. Figure 8A shows that the pigtail catheter 102 is placed directly adjacent the annular ring 2. The computer implemented method can be configured to identify and track the pigtail catheter 102, in particular a shape of the pigtail catheter 102, as a first feature to determine the position/orientation of the annular ring 2 indirectly. Alternatively, calcifications on the aortic valve/annular ring 2 may be identified and tracked as the first feature. The computer implemented method may further track the curved/coiled guidewire 101, in particular the shape of the curved/coiled guidewire 101 in the left ventricle, as a second feature. A dynamic geometric relationship between these two features can be used to determine the position of the annular ring 2 in the absence of rapid pacing. Based on a machine-learning model, this dynamic geometric relationship may be adapted to account for the reduced pressure/blood flow during rapid pacing to determine a modified position/orientation of the annular ring reliably. A computer implemented method according to Figs. 8A - 8C may be carried out without injecting contrast agent since the previously mentioned features are visible under fluoroscopy.

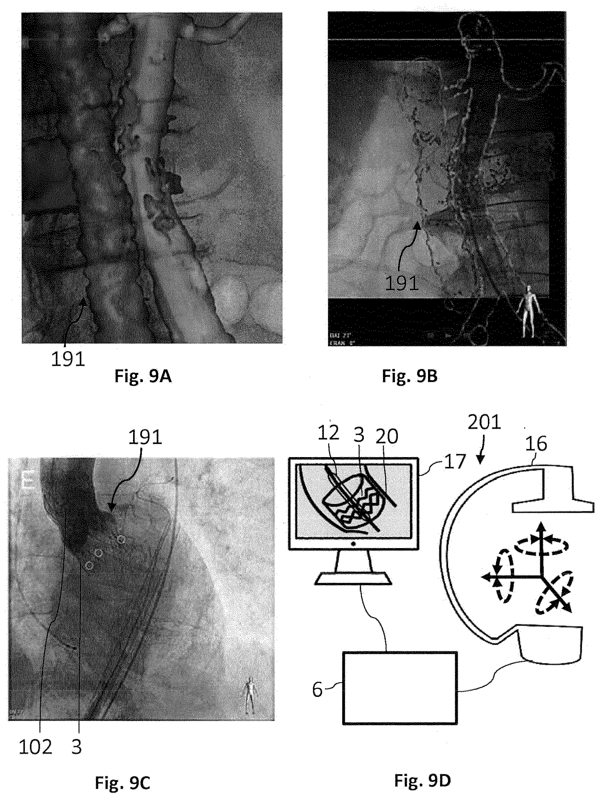

Figures 9A and 9B show a combined three-dimensional computational model 191 based on a CT image and at least two fluoroscopic images at different orientations. The three-dimensional computational model 191 is generated based on segmenting the rigid anatomy of the patient, e.g. the bones, in the CT image and the fluoroscopy images. Subsequently, the two- and three-dimensional segmentation information using the bones is projected to register the two-dimensional fluoroscopy images and the CT-image to achieve precise alignment. By using fluoroscopy images at two different orientations the accuracy of the three-dimensional computational model 191 and alignment can be enhanced.

Figure 9C shows a fluoroscopic image including the information of the three-dimensional computational model 191 of Figs. 9A and 9B. Figure 9C shows that a pigtail catheter 102 was arranged in the non-coronary cusp. A position/an orientation and a shape of the pigtail catheter 102 is detected and tracked in a plurality of fluoroscopic images as a first feature to be indicative of the position/orientation of the annular ring. The segmented bones or another anatomical or procedural feature in the images or the three-dimensional model 191 may be used as a second feature to determine a dynamic geometric relationship as previously described, such that a modified position/orientation of the annular ring during rapid pacing can be determined.

Based on these features a C-arm (see Fig. 9D) may be operated or instructions for operation may be provided to a clinician. A position/orientation of a heart valve prosthesis 3, in particular a three-dimensional representation, may then be identified, tracked and included in.the three-dimensional computational model 191. The modified position/orientation of the annular ring is then used to deploy a heart valve prosthesis 3 at a target implantation depth. Due to the three-dimensional model 191, additional information of the orientation of the heart valve prosthesis 3 are provided which allow correcting the tilt and circumferential alignment of the heart valve prosthesis 3 with respect to the annular ring.

Figure 9D shows a schematic representation of a system 201 including a data processing unit 6 for carrying out the method of determining a modified position/orientation of the annular ring during rapid pacing. The system 201 is connected to a display device 17 which displays a position/orientation of a heart valve prosthesis 3 with respect to the modified position/orientation of the annular ring in real-time.

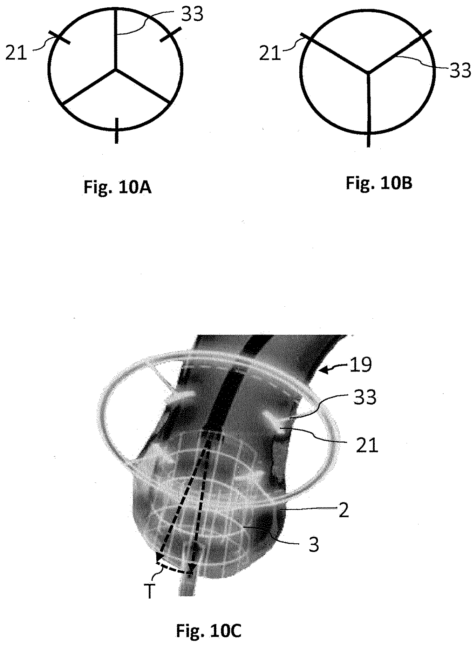

The system 201 is further adapted to display the tilt and circumferential orientation of the prosthesis 3 with respect to the ' modified position/orientation 20 of the annular ring as described in further detail in Figs. 10A - 10C. The system 201 is adapted to provide control commands to a clinician or a robotic controller to adjust the tilt, circumferential orientation of the prosthesis 3, and a longitudinal position of the prosthesis 3 to align the prosthesis 3 with the annular ring 2 during rapid pacing.

The data processing unit 6 is further adapted to operate a C-arm 16 for positioning the imaging unit for acquiring the fluoroscopy images, such that the C-arm is positioned for the optimal projection view and is moved between the anteroposterior view, the right oblique or left oblique view, and is tilted cranially or caudally in a manner known to the person skilled in the art. The C-arm is movable in 4 or more degrees of freedom. Alternatively, the data processing unit 6 merely provides user instructions on the display 17 on how to achieve the optimal projection view and operate the C-arm.

Figure 9D further shows that the data-processing unit 6 displays an expansion state 31, 32 (see Figs. 6A and 6B) of the heart valve prosthesis 3 on the display 17 and provides instructions on how to operate a balloon catheter 12 for deploying the prosthesis 3 on the display 17 in real-time. The instructions are based on a determined target radial dimension in a previously described manner (see Fig. 6A and 6B).

Figures 10A and 10B show a virtual schematic two-dimensional representation of a heart valve prosthesis and the annular ring having a visual marking 33 which is indicative of a circumferential orientation of the heart valve prosthesis 3 and a visual marking 21 which is indicative of a circumferential orientation of the aortic annular ring.

The circumferential orientation of the heart valve prosthesis may be determined by a non-rotationally symmetric radiopaque design of the medical instrument, in particular via radiopaque indicator of the heart valve prosthesis or a design of the heart valve prosthesis.

The circumferential orientation of the annular ring may be determined based on the cusps of the native aortic valve annulus in the fluoroscopic images, e.g. based on the two-cusp view.

The visual markings 33 indicative of the circumferential orientation of the heart valve prosthesis 3 were determined based on the non-contrast images with the previously described method. The visual markings 21 indicative of a circumferential orientation of the annular ring were determined based on the contrast images with the previously described method.

The orientation of these visual markings 33, 21 with respect to each other may allow for simplified circumferential alignment of the heart valve prosthesis with respect to the annular ring, in particular in respect to the cusps of the aortic valve, by a clinician or a robotic controller. The method may include the step of calculating these visual markings 33, 21 via a processing unit as previously described and displaying these visual markings 33, 21 on a display device 17 as a visual indicator (see Fig. 9D).

As shown in Fig. 10A, the visual markings 33, 21 are not aligned while Fig. 10B shows that the visual markings 33, 21 are essentially perfectly aligned which indicates a target orientation / a target position of the heart valve prosthesis. These visual markings 33, 21 may facilitate a deployment of the heart valve prosthesis in the aortic annulus. The visual markings 33, 21 are color-coded or have a specific shape to facilitate alignment of the heart valve prosthesis and the annular ring.

Figure 10C shows a schematic three-dimensional computational model 19, 191 (see Figs. 7 or 9A - 9C) of the heart valve prosthesis 3 and the aortic root which indicates a tilt of the heart valve prosthesis 3 with respect to the annular ring 2. In addition, to the previously described visual markings 33, 21 osf the heart valve prosthesis 3 which indicate the circumferential orientation of the heart valve prosthesis 3 with respect to the annular ring 2, the three-dimensional representation includes a colour coding scheme.

The colour coding scheme indicates a tilt T of the heart valve prosthesis 3 in a current medical image with respect to a target position in the annular ring 2. The colour coding scheme further indicates a longitudinal position of the heart valve prosthesis 3 with respect to the annular ring 2. Alternatively to a color-coded scheme, arrows may indicate directional information. This allows a reliable placement of the aortic valve prosthesis within the annular ring 2 and renders TAVI/TAVR procedures less demanding for clinicians without necessitating an intensive learning process. Proper placement may further improve hemodynamic, prevent paravalvular leakage, valve migration, or embolization, maximize a lifespan of the implant, and optimize the overall patient outcomes.

## Claims

1. A computer implemented method for determining a position and/or an orientation of a valve component or a valve annulus component, preferably the annular ring (2) of a heart valve, in particular during an implantation of a heart valve prosthesis (3), comprising the steps of:
- receiving at least one first image (4) and at least one second image (5) of a moving heart via an input interface, in particular in real-time,
- identifying at least one first feature (7) indicative of a position and/or an orientation of the valve component or the valve annulus component and at least one second feature (8) in the at least one first image (4) using a data processing unit (6),
- determining at least one static or dynamic geometric relationship between the at least one first feature (7) and the at least one second feature (8) based on a localization of the first and second features (7, 8) in the at least one first image (4), using the data processing unit (6), identifying the at least one second feature in the at least one second image (5) using the data processing unit (6),
- determining a modified position and/or orientation (20) of the valve component or the valve annulus component in the at least one second image (5) based on the at least one second feature (8), the at least one static or dynamic geometric relationship, and a static or dynamic geometric relationship model to account for the position and/or the orientation of the valve component or the valve annulus component, in particular during rapid pacing of the heart,
- transmitting the modified position and/or orientation (20) of the valve component or the valve annulus component to an output interface.

2. The computer implemented method according to claim 1, wherein the at least one first image (4) is a normal-flow image being acquired in the absence of rapid pacing and the second image is a reduced-flow image being acquired during rapid pacing.

3. The method according to one of the preceding claims, wherein the method comprises one of the steps of:
- identifying the presence or absence of at least one first feature (7) indicative of a position and/or orientation of the valve component or the valve annulus component in the at least one second image (5), and
- if the presence of the first feature (7) was identified, determining the modified position and/or orientation (20) of the valve component or the valve annulus component in the at least one second image (5) based on the first feature (7),
- if the absence of the first feature (7) was identified, determining the modified position and/or orientation (20) of the valve component or the valve annulus component in the at least one second image (5) only based on the at least one second feature (8) and the at least one modified static or dynamic geometric relationship and the static or dynamic geometric relationship model.

4. The method according to one of the preceding claims, wherein method comprises the step of:
- Determining a position and/or an orientation of a heart valve prosthesis (3) and/or of a delivery device for delivering the heart valve prosthesis (3) in at least one of the at least one first and/or second images (5) in real-time, in particular with respect to the modified position and/or orientation (20) of the valve component or the valve annulus component.

5. The method according to one of the preceding claims, wherein the first feature (7) in the at least one of the at least one first image (4) is indicative of a maximum radial dimension of the annular ring (2), and the method optionally comprises the further step of
determining a target radial dimension of the annular ring (2) in the at least one second image (5) based on the maximum radial dimension, in particular during rapid pacing.

6. The method according to one of the preceding claims, wherein the at least one determined static or dynamic geometric relationship is indicative of the cardiac cycle and/or the respiratory cycle of the patient, in particular a first static or dynamic geometric relationship being based on the cardiac cycle and a second static or dynamic geometric relationship being based on the respiratory cycle.

7. The method according to one of the preceding claims, wherein the at least one second feature (8) is formed by
- an anatomical feature of the patient or
- a procedural feature, in particular a medical instrument (101, 102) or a pose of the medical instrument (101, 102),
preferably one second feature (8) being an anatomical feature, and another second feature (8) being a procedural feature.

8. The method according to one of the preceding claims, wherein the method comprises the step of:
- processing, preferably in real-time, the at least one first and/or second images (4, 5) and including at least one of (i) the modified position and/or orientation (20) of the valve component or the valve annulus component and (ii) the position and/or orientation of the heart valve prosthesis (3) in one of:
o a pre-operative visualization of the heart, and
o a computational model (2) of the valve component or the valve annulus component, in particular a finite element model.

9. The method according to one of the preceding claims, wherein the method comprises the step of:
- Displaying the modified position and/or orientation (20) of the valve component or the valve annulus component and/or the heart valve prosthesis (3) in
o the at least one second image (5) or the at least one first image (4),
o the pre-operative visualization, and/or
o in the computational model..

10. The method according to claim 9, wherein the method comprises the steps of:
- receiving a user generated input-to adjust the modified position and/or orientation (20) of the valve component or the valve annulus component manually and
- displaying the adjusted modified position and/or orientation instead of the prior modified position and/or orientation (20), preferably in real-time.

11. The method according to one of the preceding claims, wherein the method comprises the step of:
- Classifying the at least one first and/or second images (4, 5) to determine, if the respective image (4, 5) was acquired under the influence of a contrast agent, and
- Determining at least one of the at least one first feature (7) and the second feature (8) which is not visible without contrast agent based on the other one of the at least one first feature (7) and the second feature (8) which is visible without contrast agent, if the image (4, 5) was not acquired under the influence of the contrast agent.

12. The method according to one of the preceding claims, wherein the step of determining the modified position and/or orientation (20) of the valve component or the valve annulus component is based on an electrocardiogram of the heart using the data processing unit (6).

13. Computer program product comprising instructions which, when the program is executed by a computer, cause the computer to carry out the steps of the method of one of the claims 1 - 12.

14. A computer-readable storage medium comprising instructions which, when executed by a computer, cause the computer to carry out the steps of the method of one of the claims 1 - 12.

15. Use of the computer implemented method of one of the claims 1 - 12 for at least one of:
- planning a transcatheter aortic valve implantation or replacement,
- translationally moving, rotationally moving, and/or tilting about a longitudinal axis at least one medical instrument (101, 102), in particular a delivery system of a heart valve prosthesis (3) or the heart valve prosthesis (3) for navigating and/or deploying the heart valve prosthesis (3), and
- optimizing an amount of contrast agent and/or a time interval of introducing the contrast agent into a body duct (11).

16. A system (201) comprising means, and in particular a data processing unit (6), adapted to execute the steps of the computer implemented method of one of the claims 1 - 12.

17. The system according to claim 16, wherein the data processing unit (6) is configured for determining a dynamic radial dimension of the heart valve prosthesis (3) or of an expansion unit (12) for expanding the heart valve prosthesis (3), in particular comprising a balloon, in real-time and
the data processing unit (6) is configured to provide
- (i) an expansion control command for operating the expansion unit (12) or
- (ii) expansion control instructions to a user to operate the expansion unit (12),
for radially expanding the heart valve prosthesis (3) in the annular ring (2), in particular until reaching at least the target radial dimension of the annular ring (2).

18. The system according to one of the claims 16 or 17, wherein the data processing unit (6) is configured for determining a deployment control command based on the modified position and/or the orientation (20) of the valve component or the valve annulus component and the position and/or the orientation of a heart valve prosthesis (3) for operating the delivery device, to deploy the heart valve prosthesis (3) in the annular ring (2), wherein the control command provides instructions on at least one of, in particular all of:
- adjusting a tilt of the heart valve prosthesis (3) with respect to the annular ring (2),
- adjusting a circumferential orientation of the heart valve prosthesis (3) with respect to the annular ring (2), and
- adjusting a longitudinal position of the heart valve prosthesis (3).

19. The system according to one of the claims 16 to 18, wherein the system comprises a pacing unit which is configured for delivering pacing signals to a target region of a patient's heart in proximity of the valve component or valve annulus component for reducing the flow across the valve, wherein the pacing unit is in particular operable by the data processing unit (6).

20. The system according to one of the claims 15 - 18, wherein the data processing unit (6) is configured for receiving at least one post-deployment image (41) after deployment of the heart valve prosthesis (3) and identifying and validating the position and/or orientation of the heart valve prosthesis (3) with respect to the first feature (7) indicative of the position and/or orientation of the valve component or the valve annulus component in the at least one first image (4).

21. The system according to one of the claims 16 - 20, wherein the data processing unit (6) is configured for:
- controlling an imaging device positioning unit (15) for positioning and/or orienting the imaging unit (16) or
- providing user instructions on how to operate the imaging device positioning unit (15), wherein
the imaging device positioning unit (15) may comprise a C-arm.

22. A system, in particular according to one of the claims 16 - 21, for guiding the placement of a heart valve prosthesis (3) within an annular ring (2), the system comprising a data processing unit (6) which is adapted for displaying on a display device (17) a position and/or orientation of a heart valve prosthesis (3) with respect to a modified orientation and/or position (20) of the valve component or the valve annulus component in real-time.

23. The system according to claim 22, wherein the data processing unit (6) is adapted for generating visual markings (33, 21), in particular color-coded visual markings, indicating the relative position and/or orientation of the heart valve prosthesis (3) and/or instructions on reaching the desired orientation and/or position of the heart valveprosthesis in the annular ring (2) in real-time based on at least one of, in particular all of, the following:
- a relative tilt (T) of the orientation of the heart valve prosthesis (3) with respect to the modified orientation of the annular ring (2),
- a relative circumferential orientation of the orientation of the heart valve prosthesis (3) with respect to the modified orientation of the annular ring (2), and
- a relative longitudinal position of the heart valve prosthesis (3) with respect to the modified position of the annular ring (2).

24. The system according to one of the claims 22 or 23, wherein the data processing unit (6) is adapted for displaying an expansion state of the heart valve prosthesis (3) or an expansion unit (12) on the display device (17) in real-time.
